# DEMANDE DE BREVET EUROPEEN

(11) **EP 0 791 657 A1**
(43) Date de publication de la demande: **27.08.1997**
(21) Numéro de dépôt: 97400409.5
(22) Date de dépôt: 25.02.1997
(51) Int. Cl.: C12P 7/40

(54) **Procédé de production naturelle d'acide formique ou de formiate**

(30) Priorité: 26.02.1996 FR 9602365
(71) Demandeur: Lesaffre Développement, 59706 Marq en Baroeul (FR)
(72) Inventeur: Loubiere, Pascal, 31000 Toulouse (FR); Lindley, Nicolas, 81310 Parisot (FR); Vidor, Emmanuel, 44230 Saint Sebastien Sur Loire (FR); Taillade, Patrick, 59420 Mouvaux (FR)
(74) Mandataire: Koch, Gustave

(57) **Abrégé**

L'invention a pour objet l'utilisation, pour la fabrication d'acide formique et/ou de formiate à partir d'un milieu de culture comportant un sucre fermentescible, d'une souche bactérienne
- présentant une déficience dans le transport ou le métabolisme d'au moins un sucre fermentescible,
- comportant une voie active de dégradation du sucre précédent par la pyruvate formiate lyase,
- capable de transformer majoritairement le sucre précédent en acide formique et/ou formiate même en présence d'une concentration non limitante dudit sucre.

Elle vise également un procédé de fermentation par culture de ladite souche en vue de la transformation d'un sucre fermentescible en acide formique ou en formiate, ainsi que les milieux de culture enrichis en acide formique ou en formiate ainsi obtenus, ainsi qu'un procédé d'extraction/purification de l'acide formique sous forme d'un de ses esters volatiles, de préférence du formiate d'éthyle.

## Description

L'acide formique ou acide méthanoïque est le premier des acides de la série aliphatique des acides carboxyliques; c'est un liquide clair, incolore, à odeur piquante et très agressif.

Il a de multiples applications dans les industries du cuir ou du textile, ou encore comme agent de conservation dans les ensilages dans lesquels il agit par inhibition de la croissance des moisissures.

Il est également utilisé comme molécule aromatique dans un grand nombre d'aliments et de boissons. Ses esters, notamment le formiate d'éthyle ont également un grand intérêt comme molécules aromatiques (FENAROLI's Handbook of Flavor Ingredients, Vol.11, 3ème édition, CRC Press, p.280 et 231).

Il est quasi-exclusivement produit par synthèse chimique. Il existe à l'état naturel; cette molécule a été découverte en 1670 parmi les produits résultant de la distillation des fourmis. On retrouve également l'acide formique dans une large variété de plantes, et il est produit comme substance aromatique naturelle pour l'industrie des arômes par extraction à partir de plantes, notamment d'orties.

Selon la réglementation des Etats-Unis d'Amérique, ou selon la réglementation européenne (Directive du Conseil n° 88-388/CEE sur les arômes), les molécules aromatiques obtenues par des procédés microbiologiques à partir d'une matière première d'origine végétale ou animale ont, elles aussi, le statut de molécules aromatiques naturelles. Il semble que l'on ne trouve pas actuellement dans le commerce de l'acide formique répondant aux normes de pureté des molécules aromatiques et préparé par un procédé microbiologique, ce qui peut s'expliquer par le fait que l'acide formique est bien connu pour avoir des propriétés antiseptiques et donc des propriétés inhibitrices des fermentations à des doses faibles (1 g/litre pour *Escherichia coli).*

L'ouvrage "Bactéries Lactiques", coordonnateurs Roissart et Lucquet, Edition Lorica, Janvier 1994, Vol.1, référence ISBN: 2-9507447-0-1, fournit une description détaillée des voies métaboliques qui conduisent les bactéries lactiques à produire soit uniquement de l'acide lactique (bactéries lactiques homofermentaires), soit de l'acide lactique, de l'acide acétique et de l'éthanol (bactéries lactiques hétérofermentaires), et parfois d'autres produits tels que l'acide formique, ces derniers étant toutefois formés en quantités mineures dans des conditions très précises de substrat limitant. Cet ouvrage peut être considéré comme un manuel de référence, largement exhaustif. La terminologie scientifique utilisée ci-après est celle de cet ouvrage, et il est renvoyé à cet ouvrage pour l'explicitation de l'ensemble des mécanismes et voies métaboliques des bactéries lactiques, en particulier aux pages 184 à 187, 242 à 257, 276 à 281, 407. Le terme "bactéries lactiques" englobe, comme décrit dans cet ouvrage, les bactéries appartenant notamment aux genres *Streptococcus, Lactococcus, Pediococcus, Lactobacillus, Bifidobacterium,* étant entendu que, contrairement au "Bergey's Manual of Systematic Bacteriology" (1986), les Lactic Acid Streptococci, à savoir *Streptococcus lactis* et *Streptococcus raffinolactis* sont appelés *Lactococcus.*

Le document WO 95/19425 décrit l'utilisation de souches bactériennes du genre *Bacillus* pour la production par voie métabolique de métabolites tels que le L(+)- lactate, ainsi que des produits autres que le L(+)- lactate, notamment l'éthanol, le formiate et l'acétate.

Dans les différents exemples et en présence d'un excès de glucose, l'acide lactique est produit majoritairement; par contre, en milieu limité en glucose et avec un large excès de facteurs de croissance coûteux (ajout de deux fois plus d'un mélange "extrait de levure/caséine trypsique" que de glucose), on obtient un milieu de fermentation ayant une concentration finale de 2,5 g/litre en acide lactique, de 5,5 g/litre en acide acétique, de 4,1 g/litre en éthanol et de 8,4 g/litre en acide formique.

La Société Demanderesse s'est donnée comme but de produire économiquement de l'acide formique naturel par voie microbiologique à des teneurs telles dans le milieu de culture qu'il devienne aisé à extraire et à purifier sous une forme répondant aux normes du "Food Chemicals Codex" (National Academy Press, USA) et de l'industrie aromatique. Elle a constaté que ce but pouvait être atteint en utilisant des souches bactériennes qu'elle a eu le mérite de sélectionner et qui sont capables de transformer un substrat fermentescible, non limitant, majoritairement en acide formique ou en formiate; elle a également mis au point un procédé mettant en oeuvre les souches bactériennes en question.

Les souches bactériennes utilisées selon l'invention pour la production d'acide formique sont caractérisées par le fait qu'elles
- présentent une déficience dans le transport ou le métabolisme d'au moins un sucre fermentescible,
- comportent une voie active de dégradation dudit sucre par la pyruvate formiate lyase,
- sont capables de transformer majoritairement ledit sucre en acide formique, même en présence d'une concentration non limitante en ledit sucre.

Les souches bactériennes en question ont la propriété surprenante de permettre l'obtention dans le milieu de culture d'une concentration finale en acide formique ou formiate supérieure à 10 g/litre.

Ces souches bactériennes sont par exemple des souches de bactéries lactiques homofermentaires déficientes dans leur système principal de transport membranaire du lactose ("Phosphotransferase System Lactose" ou encore système Lac PT/P-β-gal) et dont la fermentation n'est pas inhibée par l'acide formique jusqu'à au moins 10 g/litre d'acide formique.

Ces souches bactériennes sont de préférence des bactéries lactiques homofermentaires du genre de celles dans lesquelles le transport du lactose est principalement déterminé par les gènes qui sont portés par un ou plusieurs plasmides lactose, lesdites souches présentant la particularité de ne pas comporter le ou les plasmides en question, sans être lac⁻, c'est-à-dire sans être dans l'impossibilité d'utiliser le lactose.

Elles appartiennent notamment au genre *Lactococcus,* plus précisément à l'espèce *Lactococcus lactis.*

Un représentant de ce genre, appartenant à l'espèce *Lactococcus lactis,* variété *lactis,* est la souche déposée au N.C.D.O. (National Collection of Dairy Organisms) devenu le N.C.F.B. (National Collection of Food Bacteria), précédemment à Reading en Angleterre, et maintenant à Aberdeen, en Ecosse, à l'adresse suivante 23 St Machar Drive, Aberdeen AB2 1RY, Scotland, UK, sous le n° 2118. Cette souche est désignée ci-après comme souche N.C.D.O. n°2118.

D'un point de vue général, les souches bactériennes utilisées conformément à l'invention sont déficientes dans au moins un de leurs systèmes de transport du lactose, et très généralement dans leur système PTS ("Phosphotransferase System") lactose qui est le système prépondérant chez les bactéries lactiques, tout en ayant gardé la possibilité d'utiliser le lactose; elles comportent une voie active de dégradation du pyruvate formé à partir du lactose par la pyruvate formiate lyase et comportent une séquence d'ADN capable de s'hybrider pour au moins 70%, et de préférence au moins 80%, avec l'ADN de la souche N.C.D.O. n° 2118. Ces souches permettent une concentration finale, après culture en anaérobie sur lactose, d'au moins 10 g/litre d'acide formique.

D'un point de vue encore plus général, les souches bactériennes utilisées selon l'invention sont les souches qui, d'une part, en conditions non limitantes d'un substrat carboné, généralement un sucre fermentescible, utilisent ce substrat carboné en anaérobiose sans produire ou en produisant des quantités intracellulaires faibles de fructose diphosphate, de telle sorte que la transformation du pyruvate formé à partir de cette source de carbone en lactate soit inhibée, ou très faiblement active, et qu'au contraire le pyruvate formé soit majoritairement dégradé par la pyruvate formiate lyase en formiate, en éthanol et/ou en acétate et qui, d'autre part, permettent des productions d'au moins 10 g/litre d'acide formique dans le milieu de culture, avant d'être inhibées par l'acide formique formé.

Le procédé de fermentation conforme à l'invention pour la préparation de l'acide formique ou de formiate est caractérisé par le fait que l'on cultive au moins l'une des souches utilisées conformément à l'invention en anaérobiose sur un milieu de culture comportant, d'une part, une source de carbone constituée par un sucre fermentescible dans lequel ladite souche présente une déficience dans le transport ou dans le métabolisme et, d'autre part, une source d'azote, de préférence un extrait de levure.

Selon un mode de réalisation avantageux du susdit procédé, la concentration du milieu de culture en substrat fermentescible est de 2 à 80 g/l, de préférence de 5 à 60 g/l et plus préférentiellement de 10 à 30 g/l; sa concentration en extrait de levure est de 2 à 20 g/l, de préférence de 5 à 20 g/l et plus préférentiellement de 8 à 12 g/l.

Selon un autre mode de réalisation avantageux du susdit procédé, la source d'azote est constituée par un mélange des acides aminés essentiels à la croissance de la souche bactérienne utilisée, avec apport des complémentations nécessaires en vitamines.

Selon un autre mode de réalisation avantageux du susdit procédé, la source d'azote est constituée par des extraits de levure, par des extraits de viande, par toute peptone ou par tout hydrolysat de protéines donnant des résultats équivalents aux extraits de levure en matière de production d'acide formique.

Selon un autre mode de réalisation avantageux du susdit procédé, la température du milieu de culture est de 20 à 40°C, de préférence de 25 à 37°C et plus préférentiellement de 28 à 35°C, et son pH est de 4 à 10, de préférence de 6,5 à 7,8 et plus préférentiellement de 7 à 7,7.

Selon un mode de réalisation préféré du susdit procédé, la fermentation est réalisée en discontinu avec alimentation continue des nutriments ou "fed-batch", c'est-à-dire avec apport progressif de la source de carbone et apport complémentaire progressif de la source d'azote, de préférence constituée d'extraits de levure. Ces apports progressifs seront réalisés de manière à avoir, pendant au moins 90% du temps de la fermentation au minimum dans le milieu de culture, d'une part au moins 2 g/l de lactose et, de préférence, au moins 5 g/l de lactose et, d'autre part, au moins 2 g/l d'extrait de levure et, de préférence, au moins 5 g/l d'extrait de levure.

L'invention vise également, en tant que produits industriels nouveaux à partir desquels il est possible d'isoler l'acide formique ou le formiate, les milieux de culture susceptibles d'être obtenus par mise en oeuvre du procédé conforme à l'invention et qui sont caractérisés par une teneur en acide formique ou formiate supérieure à 10 g/l, de préférence supérieure à 20 g/l.

Ces milieux de culture sont caractérisés également par un rapport quantité présente d'acide formique ou de formiate en masse (ou en g/litre) sur quantité présente d'acide lactique en masse (ou en g/litre) supérieur à 1.

La teneur de ces milieux de culture en acide lactique ou lactate est de préférence inférieure à 10 g/l.

L'invention pourra être encore mieux comprise à l'aide du complément de description qui suit et des exemples non limitatifs qui sont relatifs à des modes de réalisation avantageux de certains aspects de l'invention.

Les inventeurs ont montré que la souche bactérienne du type *Lactococcus lactis subsp. lactis* déposée au N.C.D.O. sous le n° 2118 est la souche type à utiliser selon l'invention.

Ils ont également montré que cette souche et les souches équivalentes objets de l'invention, qui sont déficientes en PTS ("Phosphotransferase system") lactose et capables de croître notamment sur lactose en tant que substrat carboné fermentescible, produisaient préférentiellement, lorsqu'elles étaient cultivées en anaérobiose en présence de lactose en quantité non limitante, c'est-à-dire de 10 à 80 g/l, du formiate, de l'acétate et de l'éthanol.

Le rendement en formiate par rapport au lactose est de manière surprenante élevé; la concentration en formiate peut atteindre jusqu'à 30 g/l avant qu'il y ait inhibition totale des cellules de bactéries.

La présence dans le milieu de culture d'un excès d'une source d'azote constituée par des protéines hydrolysées ainsi que des vitamines et facteurs de croissance est nécessaire.

La nature de la source d'azote est importante pour optimiser la production d'acide formique. Les meilleurs résultats ont été obtenus avec des extraits de levure qui ont de plus le mérite de favoriser la croissance des bactéries et de retarder l'inhibition de leur métabolisme par le formiate formé jusqu'à des concentrations en formiate d'au moins 30 g/litre.

D'autres sources d'azote et de vitamines peuvent donner des résultats presque équivalents. De manière générale, l'homme de l'art sélectionne la peptone ou l'hydrolysat de protéines et les compléments vitaminés nécessaires en fonction de leur efficacité et de leur coût. Des mélanges d'acides aminés et de vitamines ou facteurs de croissance peuvent être envisagés, mais ils sont beaucoup trop coûteux. L'extrait de levure est parmi les sources d'azote et de facteurs de croissance, celle qui offre en principe le meilleur rapport efficacité/prix.

Une teneur en extrait de levure de 10 g/l (pour des valeurs testées de 2 à 30 g/l) apparaît comme optimale à la fois pour la croissance et pour la production de formiate. Cette teneur limitée permet des productions d'acide formique à coût compétitif.

La valeur de la concentration du milieu de culture en substrat fermentescible, notamment en lactose, est importante.

En effet, le rendement en formiate diminue de 2,26 à 1,42 moles par mole de lactose à mesure que l'on augmente la concentration initiale en lactose de 5 à 70 g/l dans un milieu contenant 10 g/l d'extrait de levure.

Parallèlement, le rendement en lactate augmente jusqu'à 0,64 mole par mole de lactose à mesure que l'on augmente la concentration initiale en lactose de 5 à 70 g/l dans un milieu contenant 10 g/l d'extrait de levure.

De manière surprenante, il a été mis en évidence que la production d'acide formique et de formiate est d'autant meilleure que, d'une part, on est en milieu riche en substances azotées et en facteurs de croissance, ce qui est notamment le cas d'un milieu contenant 10 g/litre d'extrait de levure et que, d'autre part, il y a assez de lactose pour permettre une croissance de la souche bactérienne utilisée, c'est-à-dire dans des conditions telles qu'on n'est en aucun cas en milieu limitant tant au niveau des sources d'azote et de facteurs de croissance, qu'au niveau de la source de carbone, ici le lactose. La valeur du pH du milieu de culture est également importante.

Ainsi, lorsque le pH évolue de 5,86 à 7,25, on constate une amélioration du rendement en formiate qui passe de 2,09 à 2,39 moles par mole de lactose et, au contraire, un effet négatif sur le rendement en lactate qui passe de 0,54 à 0,17 moles par mole de lactose.

La présence de sels minéraux, notamment alcalino-terreux tels que MgCl₂, par exemple à une concentration de l'ordre de 0,2 g/l est avantageuse. De manière générale, il doit être veillé à ce que le milieu ne présente aucune carence en éléments minéraux, y compris en oligo-éléments minéraux.

La température optimale pour favoriser la croissance et le rendement en formiate est de 20 à 40°C et, de préférence, de 28 à 37°C.

La fermentation en "fed-batch" par ajout progressif de substrat fermentescible, par exemple de lactose, et de source d'azote, par exemple d'extrait de levure, de telle sorte que soit maintenue une concentration d'environ 10 g/l de substrat fermentescible, notamment de lactose, et d'environ 10 g/l de source d'azote, notamment d'extrait de levure, pendant au moins 80% du temps de la fermentation, permet de prolonger la phase productive de formiate et d'atteindre des concentrations de 15 à 20 g/l en formiate.

L'apport progressif de source d'azote et de substrat fermentescible, notamment d'extrait de levure et de lactose, simultanément à une augmentation du pH de 7 à 7,7 par ajout de KOH, permet par exemple d'améliorer encore la teneur finale en formiate jusqu'à 30 g/litre et d'augmenter la productivité jusqu'à au moins 0,5 g/l/h. Un élément essentiel pour obtenir une productivité d'au moins 0,5 g/l/h et une concentration en acide formique supérieure à 20 g/l, de préférence supérieure à 25 g/l, est d'ensemencer le fermenteur de production d'acide formique en "fed-batch" avec une biomasse en phase exponentielle de croissance en quantité suffisante.

La fermentation en "fed-batch" est le mode préféré de production d'acide formique car elle permet de maintenir constamment dans le milieu de culture les conditions optimales de production d'acide formique, qui sont couplées avec la croissance de la biomasse. Elle permet de maintenir une concentration optimale d'extrait de levure et de lactose, d'avoir une régulation de pH avec évolution du pH en fonction de l'acide formique formé, la régulation du pH ayant pour effet notamment d'abaisser l'effet inhibiteur de l'acide formique.

Parallèlement, les concentrations en acides lactique et acétique sont limitées et sont de préférence au plus respectivement de 10 g/l et de 15 g/l, étant entendu que le rapport acide formique ou formiate produit sur acide lactique ou lactate produit est toujours supérieur à 1.

La production préférentielle de formiate a également été obtenue en présence d'autres substrats fermentescibles du groupe comprenant le galactose, le maltose, le xylose, le ribose et l'acide gluconique, sucres ou substrat carboné dont le transport et le métabolisme ne conduisent pas, en anaérobiose, avec les souches du même type que la souche N.C.D.O. n° 2118 et, de manière plus générale, avec les souches de bactéries lactiques à des concentrations intracellulaires en FDP (Fructose Diphosphate) suffisantes pour venir activer significativement la LDH (Lactate Deshydrogenase) conduisant à la transformation du pyruvate formé en lactate, d'où au contraire une activation de la pyruvate formiate lyase et une production de formiate.

Il a également été constaté qu'en utilisant la souche N.C.D.O. n° 2118, des concentrations élevées en d'autres substrats fermentescibles, notamment en saccharose, glucose, mannose, fructose et tréhalose, conduisent en anaérobiose à des teneurs faibles en acide formique, alors que l'acide lactique devient majoritaire. En effet, la souche N.C.D.O. n° 2118 n'est pas déficiente dans le transport ou le métabolisme de ces différents sucres et en conséquence leur entrée conduit à des concentrations intracellulaires importantes en FDP venant activer la LDH qui transforme le pyruvate formé à partir de ces sucres en lactate.

Pour la récupération de l'acide formique à partir du milieu de culture, on peut avoir recours aux moyens connus de l'homme du métier à propos de l'extraction de ces produits ou de produits équivalents à partir de milieux organiques et/ou à propos de la purification de l'acide formique obtenu par synthèse chimique, comme par exemple la distillation sous vide.

Cette extraction/purification de l'acide formique ou des sels de formiate à partir du milieu de culture en fin de fermentation est une opération très coûteuse et très délicate. L'acide formique est une molécule hydrophile rendant difficile l'extraction par des solvants autorisés pour l'extraction des arômes. La séparation de l'acide acétique d'une part, de l'acide formique d'autre part, au départ en mélange en solution dans l'eau nécessite un nombre d'étages théoriques de distillation très important.

Un autre aspect de l'invention est un procédé complet de production d'acide formique où celui-ci est extrait et purifié sous forme d'ester volatile comme le formiate d'éthyle. Ce procédé complet comporte les étapes suivantes:
- élimination des bactéries et des particules en suspension du milieu de culture,
- concentration par évaporation du milieu de culture contenant l'acide formique avec entraînement par la vapeur d'une partie de l'acide acétique présent,
- ajout d'alcool, de préférence d'origine naturelle en excès, cet alcool étant de préférence de l'éthanol,
- distillation de l'ester volatile formé, et récupération du formiate sous forme pure, celui-ci étant de préférence du formiate d'éthyle.

De préférence, on part d'un milieu de culture qu'on a laissé s'acidifier naturellement à un pH entre 4 et 5 en fin de fermentation. L'élimination des bactéries et des particules en suspension est réalisée par centrifugation, éventuellement avec l'aide d'un agent coagulant ou floculant. Le rendement de récupération de l'acide formique est d'au moins 90% et de préférence d'au moins 97%.

Le milieu de culture débarrassé de ses particules insolubles est alors concentré par une méthode d'évaporation rapide, comme l'évaporation sous vide. Du fait de la différence de constantes de dissociation entre les acides formique, acétique et lactique, au moins 70% de l'acide acétique présent et de préférence au moins 80% sont entraînés dans les condensats. Par contre au moins 90% de l'acide formique, et de préférence au moins 95% sont conservés dans le concentrat. Le concentrat obtenu aura de préférence au moins 100 g/litre d'acide formique, moins de 20 g/litre d'acide acétique et environ 40 à 50 g/litre d'acide lactique.

Ce concentrat est additionné d'éthanol naturel pur provenant de la fermentation de jus de betteraves à sucre, en excès par rapport à la quantité nécessaire pour estérifier l'acide formique, par exemple en quantité 3 fois supérieure à celle de l'acide formique.

Le mélange est alors introduit dans le bouilleur d'une colonne à distiller et porté à ébullition. Le formiate d'éthyle se retrouve en tête de colonne d'où il peut être soutiré. Ce soutirage du formiate d'éthyle déplace la réaction d'estérification vers la formation de formiate d'éthyle. Après recyclage, au moins 80% et de préférence au moins 90% de l'acide formique du mélange distillé est récupéré sous forme de formiate d'éthyle aux normes de pureté de cette molécule aromatique.

Au moins 65% et de préférence au moins 70% de l'acide formique présent dans le milieu de culture de départ est récupéré sous forme de formiate d'éthyle.

Dans les exemples 1 à 4 qui suivent, on a étudié successivement l'influence sur la culture de la souche N.C.D.O. n° 2118 (N.C.F.B. 2118)
- de la composition du milieu de culture,
- de la concentration en lactose,
- du pH,
- de la température.

Dans l'exemple 5, on a décrit une production d'acide formique en "fed-batch" selon le mode préféré de réalisation de l'invention. Dans l'exemple 6, on a décrit un essai d'optimisation de la production d'acide formique en "fed-batch" afin d'améliorer la productivité de cette production, la productivité étant un critère économique essentiel pour l'industrialisation des procédés biologiques. Dans l'exemple 7, on a décrit un mode préféré de réalisation de l'invention conduisant à la production de formiate d'éthyle naturel, répondant aux normes de pureté des molécules aromatiques.

### EXEMPLE 1

### Influence de la composition du milieu de culture

On décrit d'abord les matériels et méthodes utilisés de manière générale, puis les expériences en réacteurs et en tubes de culture menées pour étudier l'influence de la composition du milieu de culture, notamment l'influence des sources d'azote et de facteurs de croissance.

### I. Matériels et méthodes

### 11) Souche utilisée

La souche utilisée est la souche *Lactococcus lactis subsp. lactis* N.C.D.O. n° 2118. Cette souche N.C.D.O. n° 2118 ou N.C.F.B. 2118 a été redéposée selon le Traité de Budapest à la Collection Nationale de Cultures de Micro-organismes, Institut Pasteur, 25 rue du Docteur Roux, 75724 PARIS CEDEX 15, le 18 février 1997 sous le n° I-1849.

### 12) Milieux utilisés

On a retenu trois milieux de culture synthétiques, dénommés respectivement MCD, MS10 et MS14.

La composition de ces trois milieux est indiquée dans le tableau I.

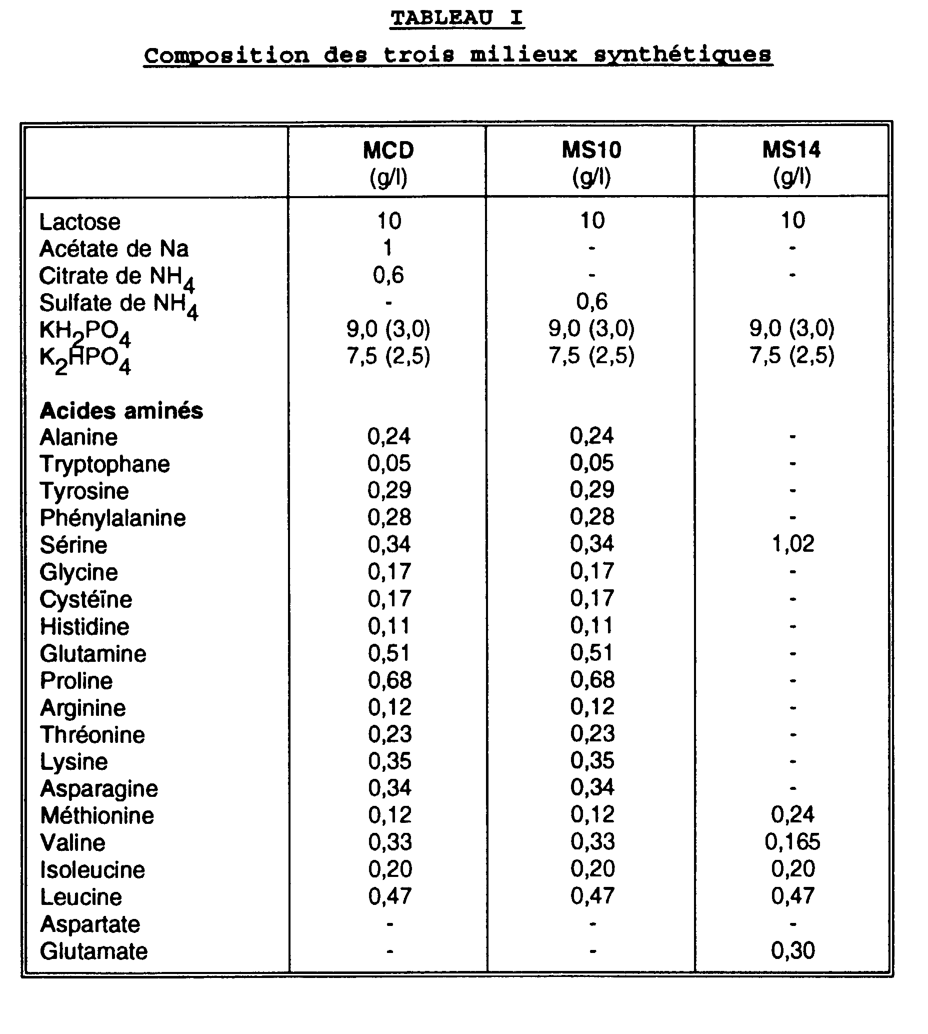

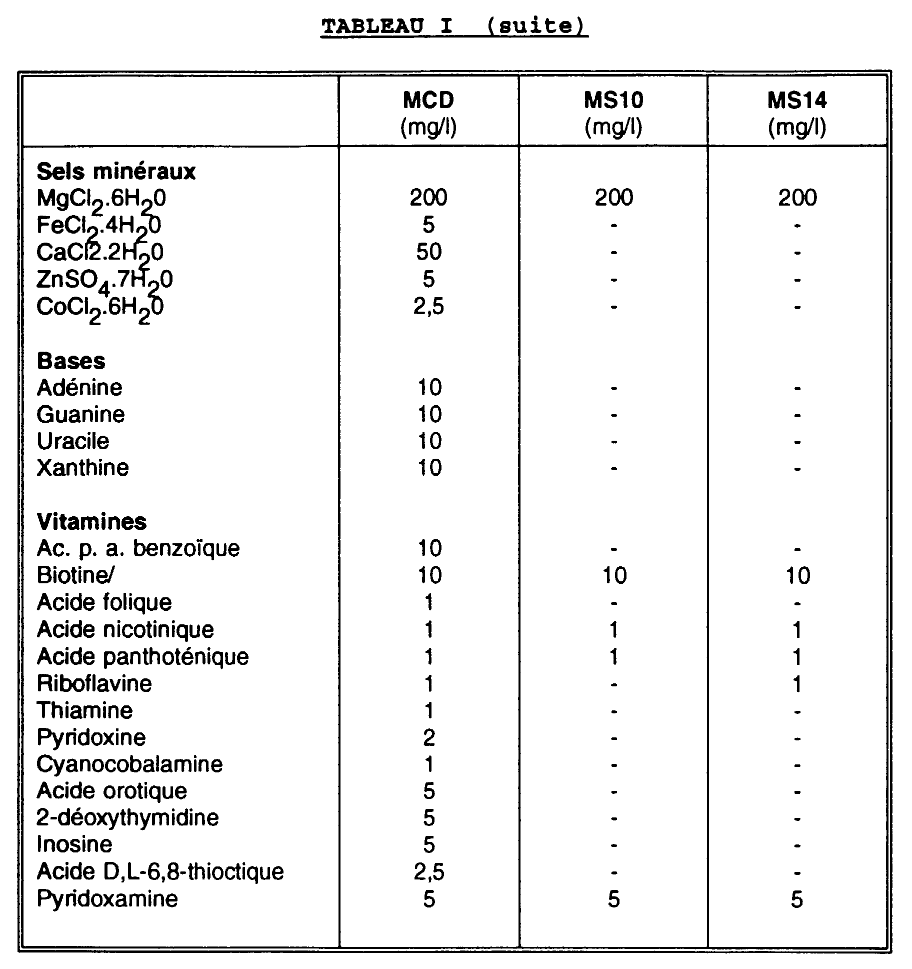

Les concentrations utilisées dans les réacteurs ou fermenteurs sont indiquées entre parenthèses dans le tableau I.

Par ailleurs, on utilise un milieu contenant un extrait de levure *Saccharomyces cerevisiae* sans sel, de haute qualité, comme par exemple l'extrait de levure BIO SPRINGER®, autolysat pur de levure *Saccharomyces cerevisiae,* en poudre sans sel, type "B", commercialisé par la Société Bio Springer, B.P. 17, 94701 Maisons-Alfort, France.

Ce milieu a la composition suivante:

| | |
|---|---|
| Extrait de levure | 10 g/l |
| Lactose | 50 g/l |
| MgCl₂, 6H₂O | 0,2 g/l |
| KH₂PO₄ | 3,0 g/l |
| K₂HPO₄ | 2,5 g/l. |

L'extrait de levure apporte tous les acides aminés, vitamines et facteurs de croissance.

### 13) Techniques de culture

### 131) Conservation de la souche

A partir d'un échantillon de la souche N.C.D.O. n° 2118, on constitue un lot de travail sur milieu MCD en tubes de culture, comme décrit ci-après.

Les cultures de la souche sont incubées à 30°C jusqu'à ce qu'elles atteignent une DO (densité optique) proche de 1,2 à 580 nm. Cela correspond à la fin de la phase exponentielle de croissance. Ces cultures sont alors mélangées à 20% de glycérol (à 80%), puis stockées à -20°C après répartition dans des cryo-tubes de marque NUNC®.

### 132) Préparation des tubes

On indique que la technique générale de préparation des tubes de préculture ou de culture consiste à utiliser des tubes en verre pour cultures anaérobies d'une contenance de 20 ml. Au préalable, ces tubes sont dégazés 10 minutes à l'azote, bouchés avec des bouchons imperméables aux gaz, sertis, puis stérilisés à 121°C pendant 20 minutes.

10 ml de chacun des milieux identifiés dans le tableau I (à pH = 6,6) sont introduits dans autant de tubes à l'aide de seringues équipées de filtres de porosité 0,2 mm, par exemple ceux de marque MINISART®.

### 133) Précultures

Avant chaque fermentation en tube, une préculture est réalisée sur le même milieu; pour ce faire, le milieu est inoculé à 4% en volume à partir d'un cryo-tube de marque NUNC® et incubé à 30°C sur table agitante jusqu'à l'obtention d'une DO optimale (0,6 sur MS14, 1,2 sur MS10 et MCD à 580 nm). A l'aide de cette préculture, on inocule à 4% en volume une seconde préculture, cette dernière étant conduite de la même manière et utilisée pour inoculer le milieu de fermentation des cultures en tubes décrites ci-dessous avec une culture en pleine phase de croissance, sans apporter d'autres éléments que ceux déjà contenus dans le milieu.

### 134) Culture en tubes

Les tubes contenant le milieu de fermentation sont donc inoculés avec 0,4 ml de préculture réalisée avec le même milieu; ils sont ensuite placés sur table agitante (120 tours/minute) à 30°C. Le suivi cinétique de la croissance est effectué toutes les heures par mesure de la DO à 580 nm à l'aide d'un spectrophotomètre à tubes, par exemple celui commercialisé sous la Société PROLABO sous la référence 320-RD.

### 135) Cultures discontinues en réacteurs agités

Les fermenteurs utilisés sont ceux commercialisés sous la marque SETRIC 2N, série SET avec virole en verre autoclavable; leur capacité utile est de 0,5 à 2 litres. L'agitation, la température et le pH sont régulés par des modules de régulation, par exemple ceux commercialisés sous la marque SETRIC.

Pendant les fermentations, les conditions sont les suivantes:
- agitation : 250 tours/minute,
- température : 30°C, maintenue par un tiroir de régulation à action proportionnelle qui commande une baffle chauffante ou une électrovanne reliée à un circuit de refroidissement,
- pH : régulé à 6,6 par une régulation proportionnelle; la sonde utilisée, par exemple celle de marque INGOLD, est préalablement étalonnée avant stérilisation, qui s'effectue montée sur le fermenteur. La solution de régulation du pH est constituée par du KOH 10 N.

Avant utilisation, le fermenteur est stérilisé avec environ 1 litre d'eau distillée à 121°C pendant 20 minutes.

Le fermenteur est ensuite équipé puis stérilisé une nouvelle fois.

L'eau est alors vidangée et une quantité de 1,6 1 du milieu défini plus haut est introduite par l'intermédiaire d'un raccord en verre relié à un tuyau en silicone.

La stérilisation du milieu s'effectue en même temps par filtration sur une cartouche de filtration, par exemple celle de marque SARTORIUS, reliée au raccord.

Ensuite, le milieu de culture est dégazé pendant 30 minutes par bullage à l'azote, l'agitation étant en marche.

Tout au long de la fermentation, une pression d'azote de 20 mbars est maintenue par un détendeur manométrique branché sur la bouteille d'azote.

Les précultures (300 ml) se font dans des bouteilles de 500 ml mais, contrairement à la procédure suivie pour les fermentations en tubes, les cellules sont concentrées lors de la deuxième préculture par centrifugation dans environ un sixième du volume initial du surnageant.

Le fermenteur est ensuite inoculé avec la préculture lavée et concentrée à l'aide d'une seringue stérile de 50 ml.

Cette opération permet d'obtenir une DO d'environ 0,2 unité lors de l'inoculation.

### II. Etudes de l'influence du milieu de culture

### 21) Expériences en réacteurs discontinus agités

Dans une première expérience en fermenteur SETRIC, on réalise une culture discontinue agitée en 10 heures avec le milieu MS10. On obtient 4 g/litre de formiate, soit un rendement en formiate de 3,24 moles par mole de lactose, très proche du rendement théorique de 4 moles d'acide formique par mole de lactose par la voie de la pyruvate formiate lyase. La production en lactate est négligeable.

Dans une seconde expérience, on utilise les fermenteurs SETRIC avec les milieux de culture suivants:
- le milieu synthétique MS14, avec 50 g/litre de lactose à la place de 10 g/litre, qui est un milieu minimal;
- le milieu synthétique MS10, avec 50 g/litre de lactose à la place de 10 g/litre, appelé milieu MS10* AA limités;
- le milieu synthétique MS10, avec 50 g/litre de lactose et parallèlement multiplication de la concentration en acides aminés par 5, appelé MS10** AA en excès;
- le milieu avec extrait de levure tel que décrit ci-dessus.

Les résultats de ces quatre fermentations sont donnés dans le tableau II.

**TABLEAU II**

| | MS14 | MS10* AA limités | MS10** AA en excès | Extrait de levure |
|---|---|---|---|---|
| Formiate mM (g/l) | 115 (5,2) | 96 (4,3) | 133 (6,0) | 255 (11,5) |
| Lactate mM (g/l) | 138,5 (12,3) | 175 (15,6) | 157 (14,0) | 79,5 (7,1) |
| Rendement formiate (mol/mol) | 0,91 | 0,74 | 1,06 | 2,04 |
| Rendement lactate (mol/mol) | 1,10 | 1,36 | 1,25 | 0,63 |
| Durée (h) | 65 | 82 | 100 | 48 |
| Productivité formiate mM/h (g/l/h) | 1,77 (0,08) | 1,17 (0,052) | 1,34 (0,06) | 5,3 (0,24) |
| Lactose consommé (g/l) | 43 | 44,1 | 43 | 42,8 |
| Extrait de levure (g/l) au départ | 0 | 0 | | 10 |
| Lactose (g/l) au départ | 50 | 50 | 50 | 50 |

Trois phases peuvent être distinguées au cours de ces quatre fermentations avec 50 g/litre de lactose dont les résultats globaux sont donnés dans le tableau II.

Une première phase qui dure une dizaine d'heures, au cours desquelles, sauf sur le milieu minimal MS14, il n'y a pratiquement que production de formiate; la raison en est que c'est seulement ou à peu près seulement la voie de la pyruvate formiate lyase qui est empruntée. Avec le milieu MS14, il y a dès le début de la culture une production de lactate. Dans cette première phase, la concentration et la productivité en formiate augmente avec la richesse du milieu en éléments nutritifs. Cette constatation montre un lien entre, d'une part, le métabolisme de la bactérie lactique et, d'autre part, la concentration et/ou la composition des acides aminés.

Une seconde phase au cours de laquelle il y a production de formiate et de lactate; cette deuxième phase s'arrête avec l'arrêt de la croissance de la biomasse.

Une troisième phase pendant laquelle, sauf avec le milieu contenant de l'extrait de levure, la production de formiate est arrêtée, seul du lactate étant produit jusqu'à l'épuisement du lactose. C'est une phase stationnaire pour la biomasse de bactéries. Sur milieu synthétique, la production de formiate doit être arrêtée pour obtenir des meilleures productivités à la fin de la seconde phase, soit au bout de 50 heures sur le milieu MS14, de 35 heures à 40 heures sur les deux milieux MS10.

On note une meilleure productivité de la production de formiate sur le milieu contenant de l'extrait de levure, d'une part, sur la durée de chacune des phases et, d'autre part, globalement sur toute la durée de la fermentation.

### 22) Cultures en tubes pour étudier différentes sources d'azote

On a déterminé par culture en tubes le taux de croissance maximum µmax(h⁻¹) en fonction de la nature de la source d'azote.

Pour ce faire, on a travaillé sur des milieux contenant tous 10 g/l de lactose et 0,2 g/l de MgCl₂,6H₂O et présentant un pH de 6,6 (amené par le tampon phosphate pour cultures en tubes), la source d'azote (présente en une quantité de 10 g/l) étant successivement constituée par
- de l'extrait de levure (ye),
- de la peptone trypsique de gélatine (ptc),
- de la tryptone (t),
- de l'extrait de viande (ev),
- de la peptone de soja (ps),
- du corn-steep atomisé (csa),
- de la peptone trysique de caséine (ptg) et
- de l'extrait de malt (me).

Dans chaque cas, on a déterminé le µmax(h⁻¹) et le rendement R en formiate et en lactate, exprimés en mol/mol.

Les résultats enregistrés sont réunis dans le tableau III.

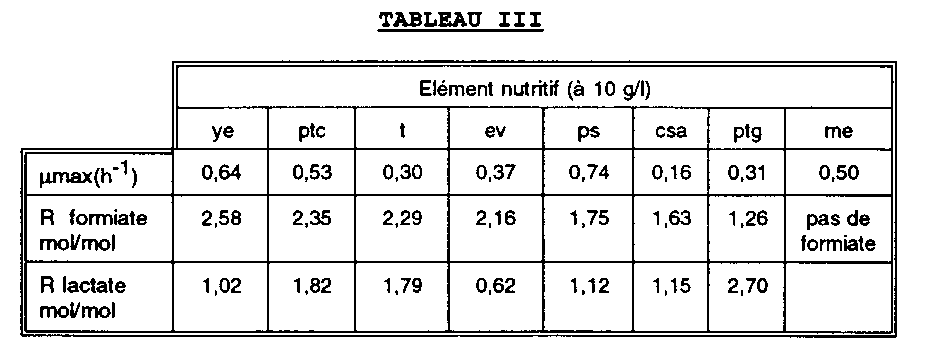

L'examen des résultats réunis dans le tableau III permet de constater que l'extrait de levure favorise la croissance et permet le meilleur rendement en formiate; il confirme l'importance de la source d'azote et des facteurs de croissance. Ces résultats montrent que l'homme de l'art devra sélectionner avec soin sa source d'azote et de facteurs de croissance.

Avec le même milieu à 10 g/litre de lactose, on a fait varier dans des cultures en tubes la concentration en extrait de levure de 2 à 30 g/litre; cette concentration est optimale, pour d'une part le taux de croissance et d'autre part le rendement mole de formiate/mole de lactose, entre 5 à 20 g/litre et plus particulièrement à environ 10 g/litre, concentration pour laquelle on obtient le meilleur taux de croissance et le meilleur rendement.

### EXEMPLE 2

### Influence de la concentration en lactose

Le milieu de culture utilisé est celui comportant 10 g/litre d'extrait de levure et décrit à l'exemple 1, à la différence près que la concentration en lactose est fixée successivement à 5, 10, 20, 30, 50, 70 et 100 g/l.

La technique de culture utilisée est la technique de culture en tubes décrite dans l'exemple 1.

Pour chacune des concentrations en lactose, on détermine:
- le taux de croissance maximal ou µmax par heure,
- le rendement en formiate exprimé en moles de formiate par mole de lactose,
- le rendement en lactate exprimé en moles de lactate par mole de lactose.

Les résultats obtenus sont réunis dans le tableau IV.

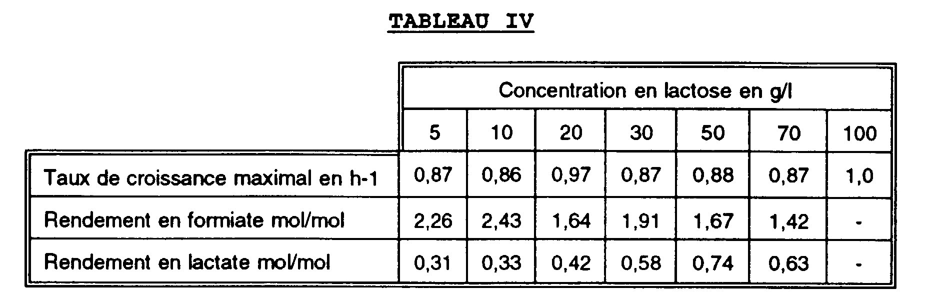

A l'examen des valeurs trouvées et réunies dans le tableau IV, on constate que le taux de croissance varie peu avec la concentration en lactose, que la concentration en formiate est prépondérante par rapport à la concentration en lactate jusqu'à 70 g/l et que le rendement en formiate est maximum à 10 g/l de lactose.

### EXEMPLE 3

### Influence du pH

On utilise le milieu de culture à base de 10 g/litre d'extrait de levure décrit dans les exemples précédents, la teneur en lactose étant de 10 g/litre.

La technique de culture est la technique de culture en tubes décrite dans l'exemple 1.

On détermine pour huit valeurs de pH, à savoir 5,86 - 6,15 - 6,5 - 6,86 - 7 - 7,25 - 7,57 et 8, le taux de croissance maximal par heure et le rendement en formiate et en lactate exprimés en moles par mole de lactose.

Les valeurs trouvées sont réunies dans le tableau V.

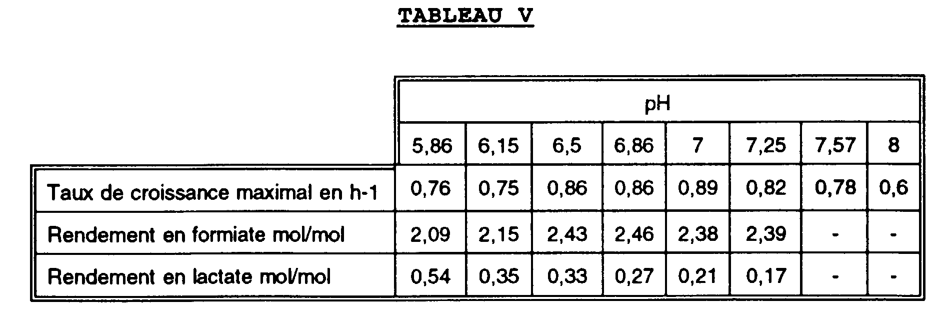

A l'examen des valeurs réunies dans le tableau V, on constate que le pH optimum de croissance se situe à 7,0, que le rendement en formiate est favorisé par un pH > 6,5 et que le rendement en lactate diminue avec l'augmentation du pH.

### EXEMPLE 4

### Influence de la température

On utilise le milieu de culture contenant 10 g/litre d'extrait de levure, 10 g/litre de lactose, 0,2g/litre de MgCl₂ et le tampon phosphate donnant un pH de 6,6 pour cultures en tubes, déjà employé dans les exemples précédents et la technique de culture en tubes décrite dans l'exemple 1.

On détermine le taux maximal de croissance par heure et les rendements (en mol/mol) en formiate et en lactate.

Les résultats sont réunis dans le tableau VI.

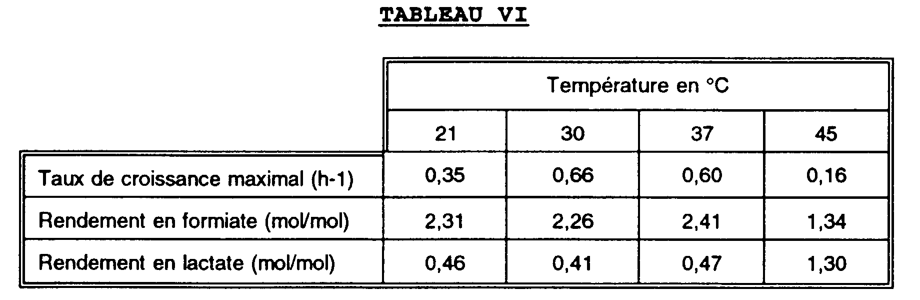

A l'examen des valeurs réunies dans le tableau VI, on constate que la température optimale est de 30 à 37°C. La température de 30°C utilisée généralement dans les différents exemples est optimale pour la croissance tout en préservant un bon rendement en formiate.

### EXEMPLE 5

### Cultures en "fed-batch" en fermenteurs agités

Par rapport aux cultures en "batch" décrites dans les exemples précédents où le milieu de culture est en totalité ajouté avant le départ de la fermentation, la culture en "fed-batch" consiste à ajouter les différents éléments du milieu de culture d'une part au départ, puis en continu ou en semi-continu de manière à maintenir dans le milieu en fermentation les concentrations jugées optimales, d'une part, pour la source de carbone, ici le lactose et d'autre part, pour la source d'azote et de facteurs de croissance, ici l'extrait de levure.

Les fermentations en "fed-batch" en anaérobiose sont conduites sur les fermenteurs de marque SETRIC décrits dans l'exemple 1 selon les conditions opératoires décrites dans l'exemple 1, à la différence des conditions spécifiques suivantes correspondant aux cultures en "fed-batch".

Le milieu de culture initial est le suivant, avant ensemencement par la biomasse de la souche N.C.D.O. n° 2118:

| | |
|---|---|
| Extrait de levure initial | 10 g/l |
| Lactose initial | 30 g/l |
| pH initial | 7 |
| MgCl₂, 6H₂O | 0,2 g/l |
| KH₂PO₄ | 1,67 g/l |
| K₂HPO₄ | 3,33 g/l |
| Température | 30°C. |

Dans le cadre du principe de la culture en "fed-batch", les additions d'extrait de levure sont faites en utilisant une solution d'extrait de levure à 300 g/litre et celles de lactose en utilisant une solution de lactose à 500 g/litre.

Le pH est régulé de 7 à 7,7, avec montée régulière par paliers de 0,2 unité par ajout de KOH.

L'extrait de levure est ajouté en continu ou en semi-continu après 10 heures de fermentation, de manière à maintenir une croissance maximale de la biomasse de bactéries le plus longtemps possible grâce à une concentration minimale équivalente en acides aminés assimilables et de facteurs de croissance apportés par l'extrait de levure d'au moins 2 g/litre et de préférence d'au moins 5 g/litre d'extrait de levure dans le milieu de culture.

Cet ajout d'extrait de levure est arrêté quand la biomasse passe en phase stationnaire.

L'alimentation en lactose commence dès que le lactose résiduel est de 5 g/l, et la concentration en lactose est maintenue à 5 g/l jusqu'à la fin de la fermentation.

La durée de l'expérience est de 42 heures.

Les résultats des mesures effectuées au bout de 42 heures sont réunis dans le tableau VII.

**TABLEAU VII**

| | |
|---|---|
| Formiate (g/l) | 22 |
| Rendement formiate mol/mol de lactose | 2,93 |
| Lactate (g/l) | 8,1 |
| Rendement lactate mol/mol de lactose | 0,55 |
| Acétate (g/l) | 13,5 |
| Rendement acétate mol/mol de lactose | 1,37 |
| Ethanol (g/l) | 10,6 |
| Rendement éthanol mol/mol de lactose | 1,40 |
| Biomasse en extrait sec (g/l) | 5,52 |

A l'examen des résultats réunis dans le tableau VII, on constate que la fermentation en "fed-batch" permet d'obtenir un très bon rendement en formiate, proche de 3 moles de formiate par mole de lactose, et une concentration élevée, supérieure à 20 g/litre de formiate.

### EXEMPLE 6

### Optimisation de la culture en "fed-batch" et extrapolation du procédé à échelle plus importante

L'amélioration de la productivité est un facteur essentiel à la rentabilité d'un procédé biochimique. Les paramètres ayant une action sur la productivité sont les profils d'apports des substrats, le maintien de conditions optimales de fonctionnement du biocatalyseur, la quantité de biocatalyseur, c'est-à-dire la quantité de biomasse de la souche N.C.D.O. 2118.

Les fermentations sont ici réalisées en mode "fed-batch" en anaérobiose sur des fermenteurs de marque SETRIC décrit dans l'exemple 1, dans les conditions de fonctionnement identiques à celles décrites dans l'exemple 5, reproduites dans l'essai 1 qui sert de référence, à la différence des conditions spécifiques suivantes portant notamment sur la quantité de biocatalyseur mise en oeuvre et la composition du milieu de préculture du biocatalyseur.

L'essai 2 a été réalisé en modifiant seulement le milieu de préculture alors que l'essai 3 est réalisé avec le milieu de préculture modifié et une augmentation de la quantité de l'ensemencement.

Le milieu de préculture pour la production de biomasse lors des essais 2 et 3 est celui décrit dans le tableau VIII ci-après.

La préculture ou production de la biomasse d'ensemencement est réalisée en mode batch ou discontinu dans les mêmes conditions que celles décrites dans l'exemple 1, le pH du milieu de préculture étant réglé à 7 dans les 3 essais.

**TABLEAU VIII**

| **Milieu de préculture des essais 2 et 3** | |
|---|---|
| Composants | % |
| Peptone (Oxoid®) | 1 |
| Lemco (Oxoid®) | 0,8 |
| Extrait de levure | 0,3 |
| KH₂PO₄ | 0,25 |
| K₂HPO₄ | 0,25 |
| MgSO₄ 7H₂O | 0,02 |
| MnSO₄ 4H₂O | 0,005 |
| Glucose | 0,5 |
| Lactose | 0,5 |

La peptone OXOID® et l'extrait de viande LEMCO OXOID® sont fournis par la Société UNIPATH, F-69572 DARDILLY.

La culture est réalisée en mode "fed-batch" dans les mêmes conditions que celles de l'exemple 5, à la différence près que la concentration initiale en lactose a été réduite à 25 g/l contre 30 g/l. L'ensemencement issu de la préculture est en phase exponentielle de croissance et représente en volume une proportion de 4% (essais 1 et 2) ou 16% (essai 3) du volume final du milieu de culture.

Les additions de solution concentrée de lactose et d'extrait de levure sont identiques à celles de l'exemple 5.

La productivité horaire est calculée en divisant la concentration de formiate obtenue par la durée de fermentation.

Les résultats obtenus sont ceux du tableau IX ci-après:

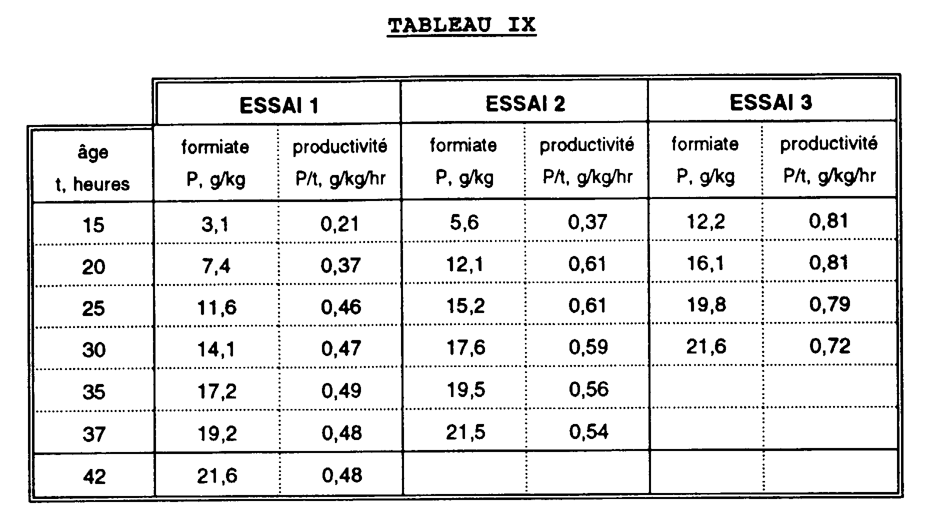

De la comparaison des essais 1 et 2 on déduit une amélioration de 20% de la productivité en acide formique ou formiate due à une biomasse d'ensemencement en phase exponentielle favorisée par un milieu de préculture plus riche.

De la comparaison des essais 1 et 3, on note un doublement de la productivité moyenne passant de 0,4-0,5 g/kg/h à 0,8-0,9 g/kg/h de formiate. Dans tous les essais, la concentration finale de formiate est supérieure à 20 g/l. La durée de la fermentation est respectivement de 42 heures pour l'essai 1, 37 heures pour l'essai 2, et 30 heures pour l'essai 3.

Les améliorations de productivité obtenues permettent une plus grande production avec une même capacité de fermentation.

Ces essais ont été confirmés en fermenteurs de 8 litres utiles puis selon des schémas où la biomasse d'ensemencement était produite dans un premier fermenteur de 8 litres en discontinu (ou "batch") pour ensemencer un second fermenteur où la production d'acide formique est réalisée en "fed-batch".

La production de biomasse d'ensemencement (c'est-à-dire du biocatalyseur) est réalisée avec une régulation du pH de manière à ce que celui-ci soit maintenu à pH 7 qui correspond à l'optimum de croissance de la bactérie, le pH pouvant être régulé indifféremment par de la soude ou de la potasse. Les transferts de culture doivent toujours être réalisés avec des biomasses en phase exponentielle de croissance et en anaérobiose stricte.

### EXEMPLE 7

### Extraction de l'acide formique par estérification - production de formiate d'éthyle

En fermenteur de 20 litres, on réalise une production d'acide formique comme décrite dans l'exemple 6. On arrête la régulation du pH en fin de fermentation de manière à ce que le milieu de culture s'acidifie naturellement. On obtient un milieu de culture ayant la composition acide suivante :

| | |
|---|---|
| Acide formique | 20-22 g/l |
| Acide acétique | 15 g/l |
| Acide lactique | 7-14 g/l (de préférence moins de 10 g/l) |
| pH | 4,5 |

3 kg de moût de fermentation sont mis en oeuvre.

### 1) Centrifugation

Cette opération est réalisée sur une centrifugeuse de laboratoire de marque JOUAN®. Pour améliorer la séparation des bactéries et des autres éventuelles matières solides en suspension, on utilise de préférence un coagulant, comme du Laviron® N Spécial fourni par la Société les Produits chimiques du Sidobre Sinnova, F-77981 Saint-Fargeau-Ponthierry, à raison de 10 g/l de moût.

Le surnageant obtenu a un poids de 2900 g.

Le rendement de récupération de l'acide formique sur ces opérations est de 97% dans le surnageant.

### 2) Concentration du moût :

La quantité de surnageant mis en oeuvre est de 2900 g.

La concentration du surnageant se fait par évaporation flash sous vide sur un équipement de laboratoire de marque HEIDOLPH OB 2001® et de capacité évaporatoire 0.5 l/h d'eau évaporée. Le surnageant est au maximum chauffé à 50°C (P = 50 mbar).

Du fait de la différence des constantes de dissociation des acides formique et acétique, il est possible, en fonction du pH, d'entraîner préférentiellement l'acide acétique par rapport à l'acide formique dans les condensats.

70% de l'acide acétique est retrouvé dans les condensats, alors que plus de 90% de l'acide formique est retrouvé dans le moût concentré.

Le surnageant concentré obtenu a la composition moyenne suivante :

| | |
|---|---|
| Acide formique | 100 g/l |
| Acide acétique | 20-25 g/l |
| Acide lactique | 50-80 g/l |

Le rendement de récupération de l'acide formique pour cette étape est de 90%.

Le surnageant obtenu a un poids d'environ 500 g.

### 3) Estérification - distillation du formiate d'éthyle

Le poids de solution mise en oeuvre dans cette étape est de 500 g.

Cette solution est mélangée à de l'éthanol. La quantité d'éthanol est trois fois supérieure à la quantité d'acide formique présent dans le surnageant concentré. On a donc rajouté 30 g d'éthanol par 100 g de surnageant. Le mélange est introduit dans le bouilleur d'une colonne à distiller, mis en chauffe jusqu'à ébullition.

Le formiate d'éthyle formé étant très volatil (température d'ébullition = 54°C), celui-ci se retrouve en tête de colonne. Lorsque la température de tête de colonne se stabilise autour de 52-53°C, le formiate d'éthyle est soutiré. Le soutirage est ajusté de façon à maintenir la température de tête de colonne constante.

Le fait de soutirer le formiate d'éthyle au fur et à mesure de sa formation permet de déplacer l'équilibre de la réaction d'estérification, et d'atteindre un rendement de 90%.

Le formiate d'éthyle obtenu a les caractéristiques moyennes suivantes:

| | |
|---|---|
| Formiate d'éthyle | 98% |
| Teneur en eau | 1 - 2% |
| Ethanol | traces |
| Acétate d'éthyle | traces |

Le rendement de distillation du produit pur est de l'ordre de 70%.

La fraction intermédiaire, riche en formiate d'éthyle et éthanol qui reste au fond du bouilleur, peut être recyclée lors des estérifications suivantes. Avec les recyclages, on récupère sous forme de formiate d'éthyle environ 90% de l'acide formique mis en oeuvre.

Ces exemples non limitatifs montrent que l'invention conduit à la production d'acide formique et de formiates, notamment de formiate d'éthyle, qui ont le statut de molécules aromatiques naturelles selon la réglementation des Etats-Unis d'Amérique, à savoir le "Code of Federal Regulations 21 Food and Drugs", ou selon la réglementation européenne, à savoir la Directive du Conseil n° 88-388/CEE sur les arômes, et les normes du "Food Chemicals Codex".

## Revendications

1. Utilisation d'une souche bactérienne caractérisée par le fait qu'elle
- présente une déficience dans le transport ou le métabolisme d'au moins un sucre fermentescible,
- comporte une voie active de dégradation du sucre précédent par la pyruvate formiate lyase,
- est capable de transformer majoritairement le sucre précédent en acide formique et/ou formiate même en présence d'une concentration non limitante dudit sucre,
pour la fabrication d'acide formique et/ou de formiate à partir d'un milieu de culture comportant ledit sucre fermentescible.

2. Utilisation selon la revendication 1, d'une souche bactérienne caractérisée par le fait qu'elle permet d'atteindre dans le milieu de culture une concentration finale en acide formique et/ou en formiate supérieure à 10 g/l.

3. Utilisation selon l'une des revendications 1 et 2 d'une souche bactérienne caractérisée par le fait qu'il s'agit d'une bactérie lactique homofermentaire du genre de celles dans lesquelles le transport du lactose est déterminé de manière prépondérante par les gènes qui sont portés par un ou plusieurs plasmides lactose, ladite souche présentant la particularité de ne pas comporter le ou les plasmides en question, tout en ayant toujours la possibilité d'utiliser le lactose.

4. Utilisation selon l'une des revendications 1 à 3 d'une souche bactérienne caractérisée par le fait qu'elle appartient au genre *Lactococcus,* plus précisément à l'espèce *Lactococcus lactis.*

5. Utilisation selon l'une des revendications 1 à 4 d'une souche bactérienne appartenant à l'espèce *Lactococcus lactis,* variété *lactis* et déposée au N.C.D.O. (National Collection of Dairy Organisms) sous le n° 2118.

6. Utilisation selon l'une des revendications 1 à 5 d'une souche bactérienne caractérisée par le fait qu'elle est déficiente dans au moins un de ses systèmes de transport du lactose, et de préférence dans son système PTS ("Phosphotransferase system") lactose, comporte une voie active de dégradation du pyruvate formé à partir du lactose par la pyruvate formiate lyase et comporte une séquence d'ADN capable de s'hybrider pour au moins 70%, et de préférence au moins 80%, avec l'ADN de la souche N.C.D.O. n° 2118.

7. Procédé de production d'acide formique ou de formiate caractérisé par le fait que l'acide formique est produit par fermentation avec une transformation majoritaire en acide formique ou en formiate d'un sucre fermentescible par culture de la souche utilisée selon l'une des revendications 1 à 6, en anaérobiose sur un milieu de culture comportant, d'une part, une source de carbone constituée par un sucre fermentescible dans lequel ladite souche présente une déficience dans le transport ou dans le métabolisme et, d'autre part, une source d'azote constituée de préférence par un extrait de levure.

8. Procédé selon la revendication 7, caractérisé par le fait que la concentration du milieu de culture en substrat fermentescible est de 2 à 80 g/l, de préférence de 5 à 60 g/l et plus préférentiellement de 10 à 30 g/l, sa concentration en extraits de levure étant de 2 à 20 g/l, de préférence de 5 à 20 g/l et plus préférentiellement encore de 8 à 12 g/l.

9. Procédé selon l'une des revendications 7 et 8, caractérisé par le fait que la source d'azote est constituée par un extrait de levure, par un extrait de viande, par une peptone ou par un hydrolysat de protéines donnant, pour la production d'acide formique, des résultats équivalents à l'extrait de levure.

10. Procédé selon l'une des revendications 7 à 9, caractérisé par le fait que la température du milieu de culture est de 20 à 40°C, de préférence de 25 à 37°C et plus préférentiellement de 28 à 35°C, et son pH est de 4 à 10, de préférence de 6,5 à 7,8 et plus préférentiellement de 7 à 7,7.

11. Procédé selon l'une des revendications 7 à 10, caractérisé par le fait que la production d'acide formique ou de formiate est réalisée en "fed-batch".

12. Procédé selon la revendication 11, caractérisé par le fait que les apports progressifs du milieu de culture pendant la production d'acide formique en "fed-batch" sont réalisés de manière à avoir, pendant au moins 90% du temps de fermentation, au minimum dans le milieu de culture, d'une part au moins 2 g/l de lactose et de préférence au moins 5 g/l de lactose et, d'autre part au moins 2 g/l d'extrait de levure et de préférence au moins 5 g/l d'extrait de levure.

13. Procédé selon la revendication 12, caractérisé par le fait que les concentrations maintenues pendant au moins 80% du temps de la fermentation sont d'environ 10 g/l de substrat fermentescible, notamment de lactose et de 10 g/l de source d'azote, notamment d'extrait de levure.

14. Milieu de culture susceptible d'être obtenu par l'utilisation selon l'une des revendications 1 à 6 ou par mise en oeuvre du procédé selon l'une des revendications 7 à 13 et caractérisé par le fait qu'il présente une teneur en acide formique ou formiate supérieure à 10 g/l, de préférence supérieure à 20 g/l.

15. Milieu de culture selon la revendication 14 caractérisé par le fait que le rapport quantité d'acide formique ou de formiate sur quantité d'acide lactique ou de lactate est supérieur à 1.

16. Milieu de culture selon la revendication 14 ou la revendication 15, caractérisé par le fait qu'il présente une teneur en acide lactique inférieure ou égale à 10 g/l.

17. Procédé selon l'une des revendications 7 à 13, à partir d'un milieu de culture selon l'une des revendications 14 à 16 caractérisé par le fait que l'acide formique est extrait ou purifié sous forme d'un ester volatile, de préférence du formiate d'éthyle.

18. Procédé selon la revendication 17 caractérisé par le fait qu'il comporte les étapes suivantes :
- élimination des bactéries et des particules en suspension du milieu de culture,
- concentration par évaporation du milieu de culture avec entraînement par la vapeur d'une partie de l'acide acétique présent,
- ajout d'alcool, de préférence de l'éthanol naturel en excès,
- distillation de l'ester volatile formé, et récupération du formiate obtenu sous forme d'un ester pur, celui-ci étant de préférence du formiate d'éthyle.

19. Procédé selon la revendication 18, caractérisé par le fait que :
- le milieu de culture de départ est de préférence à pH entre 4 et 5,
- la séparation des particules insolubles est réalisée par centrifugation, avec de préférence l'emploi d'un agent floculant,
- la concentration est réalisée par évaporation sous vide,
- le formiate d'éthyle non récupéré lors de la première distillation est recyclé, de manière à récupérer sous forme de formiate d'éthyle au moins 65% et de préférence 70% de l'acide formique présent dans le milieu de culture.

20. Acide formique susceptible d'être obtenu par le procédé selon l'une des revendications 7 à 13.

21. Formiate d'éthyle susceptible d'être obtenu par le procédé selon l'une des revendications 17 à 19.
